(19)

Europäisches Patentamt
European Patent Office
Office européen des brevets

(11) **EP 4 300 514 A1**

(12) **EUROPEAN PATENT APPLICATION**

(43) Date of publication:
**03.01.2024 Bulletin 2024/01**

(21) Application number: **23182770.0**

(22) Date of filing: **30.06.2023**

(51) International Patent Classification (IPC):
*G16H 50/30* (2018.01)　　*A61B 5/145* (2006.01)
*A61B 5/00* (2006.01)　　*A61M 5/172* (2006.01)
*G16H 20/17* (2018.01)　　*G16H 40/60* (2018.01)
*G16H 50/20* (2018.01)

(52) Cooperative Patent Classification (CPC):
**A61B 5/14532; A61B 5/7275; A61B 5/746;
G16H 20/17; G16H 40/60; G16H 50/20;
G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.07.2022　US 202263357690 P**

(71) Applicant: **Insulet Corporation
Acton MA 01720 (US)**

(72) Inventors:
• **O'CONNOR, Jason
　Acton, MA (US)**
• **LEE, Joon Bok
　Acton, MA (US)**
• **NELSON, Graeme
　Oakland, CA (US)**

(74) Representative: **Peterreins Schley
Patent- und Rechtsanwälte PartG mbB
Hermann-Sack-Straße 3
80331 München (DE)**

(54) **SYSTEM AND METHOD FOR EVALUATING RISK OF HYPOGLYCEMIA OR HYPERGLYCEMIA**

(57)　　Disclosed herein is a method, implemented in an automated drug delivery system for determining a risk of a hypoglycemic or hyperglycemic condition based on risk factors calculated each time a new blood glucose reading is received and providing an alert to the user should a risk be determined. The risk factors may include the current CGM reading, the trend of CGM readings, the amount of insulin-on-board of the user and the accuracy of previous alerts provided to the user. The method may be modified to provide the risk of hypoglycemia if the user were to engage in exercise or other physical activities.

FIG. 1

**Description**

## BACKGROUND

Many conventional automated drug delivery (ADD) systems are well known, including, for example, wearable drug delivery devices. The drug delivery device can be designed to deliver any type of liquid drug to a user. In specific embodiments, the drug delivery device can be, for example, an OmniPod® drug delivery device manufactured by Insulet Corporation of Acton, Massachusetts. The drug delivery device can be a drug delivery device such as those described in U.S. Pat. No. 7,303,549, U.S. Pat. No. 7,137,964, or U.S. Pat. No. 6,740,059, each of which is incorporated herein by reference in its entirety.

Users of ADD systems and other insulin pumps utilize information from a wide range of sources to determine their risk of hyperglycemia or hypoglycemia. These sources include, but are not limited to, blood glucose readings from a continuous glucose monitor, previous insulin delivery history, insulin-on-board values, and other datasets.

Determining a risk from an evaluation of the myriad of data available represents a significant burden for users every time it is necessary to determine whether insulin delivery or glucose ingestion is needed. Moreover, manual review of these information sources is deficient and prone to error because the user does not know precisely what actions the ADD system will take, thus significantly increasing the likelihood of missing impending risks of hyperglycemia or hypoglycemia until the risks are actually realized.

## SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

An ADD system may have access to sufficient information that is necessary to determine the user's risk of hyperglycemia or hypoglycemia. Automatically determining such a risk through an analysis of the available data streams improves response to impending risks, as opposed to relying on inaccurate manual assessments performed by the user which do not take into account all of the data available to the ADD system.

Therefore, in one aspect of the invention, disclosed herein is a method for automatically determining the user's risk of hyperglycemia or hypoglycemia based on risk factors calculated using data available in an ADD system. The method may be performed by an ADD system of the type described herein. The method may utilize population-based risk thresholds or may use risk thresholds personalized to the individual user.

In a second aspect of the invention, once the risk of either hyperglycemia or hypoglycemia is determined, which may be performed on a real-time basis, the user may be provided an indication of the risk. A real-time indicator of the risk may be generated and presented to the user, for example, via a user interface of the ADD system. In addition, recommendations may be made for actions that may be taken by the user to mitigate the risk.

In a third aspect of the invention, a method is disclosed for automatically, or at the user's request, predicting the user's risk of hyperglycemia or hypoglycemia, given a particular activity, for example, exercise. A real-time indicator of the risk, given the specific activity, may be generated and presented to the user. In addition, recommendations may be made for actions that may be taken by the user to mitigate the risk, should the user decide to engage in the specific activity.

In a fourth aspect of the invention a machine learning model may be provided which is trained to provide weighting factors for the risk factors used in the evaluation of the risk. The machine learning model may be trained using ground truth (i.e., actual) occurrences of hypoglycemia or hyperglycemia to determine a correlation between a risk factor and an actual occurrence.

## BRIEF DESCRIPTION OF THE DRAWINGS

In the drawings, like reference characters generally refer to the same parts throughout the different views. In the following description, various embodiments of the present invention are described with reference to the following drawings, in which:

> **FIG. 1** illustrates a functional block diagram of an exemplary system suitable for use with the methods disclosed herein.
>
> **FIG. 2** is a flowchart showing the process for calculating the risk of either hypoglycemia or hypoglycemia.
>
> **FIGS. 3**(**a-c**) show various examples of user interface elements utilizing text flags to provide alerts of a risk of a hypoglycemic or hyperglycemic condition.
>
> **FIGS. 4**(**a-c**) show various examples of modal windows used to provide recommendations and alerts of a risk of a hypoglycemic or hyperglycemic condition.

**FIGS. 5(a-c)** show various examples of user interface elements provide a warning of a risk of a hypoglycemic condition in the event that the user wishes to engage in a particular activity, for example, exercise.

## DETAILED DESCRIPTION

This disclosure presents various systems, components and methods for moving a liquid drug from a liquid reservoir in a drug delivery device to a patient interface, such as a needle or cannula. The embodiments described herein provide one or more advantages over conventional, prior art systems, components and methods, as further described herein.

Various embodiments of the present invention include systems and methods for delivering a medication to a user using a drug delivery device (sometimes referred to herein as a "pod"), either autonomously, or in accordance with a wireless signal received from an electronic device. The medication can be any drug in liquid form capable of being administered by a drug delivery device via a subcutaneous cannula, including, for example, insulin, GLP-1, pramlintide, morphine, blood pressure medicines, chemotherapy drugs, fertility drugs, arthritis drugs, or the like, or co-formulations of two or more of GLP-1, pramlintide, and insulin. In various embodiments, the electronic device may be a user device comprising a smartphone, a smart watch, a smart necklace, a module attached to the drug delivery device, the drug delivery device itself, or any other type or sort of electronic device that may be carried by the user or worn on the body of the user and that executes an algorithm that computes the times and dosages of delivery of the medication.

For example, the user device may execute an "artificial-pancreas" (AP) algorithm that computes the times and dosages of delivery of insulin. The user device may also be in communication with a sensor, such as an analyte sensor, for example a glucose sensor or a continuous glucose monitor (CGM), that collects data on a physical attribute or condition of the user, such as a glucose level. The sensor may be disposed in or on the body of the user and may be part of the drug delivery device or may be a separate device.

Alternatively, the drug delivery device may be in communication with the sensor in lieu of or in addition to the communication between the sensor and the user device. The communication may be direct (if, e.g., the sensor is integrated with or otherwise a part of the drug delivery device) or remote/wireless (if, e.g., the sensor is disposed in a different housing than the drug delivery device). In these embodiments, the drug delivery device contains computing hardware (e.g., a processor, memory, firmware, etc.) that executes some or all of the algorithm that computes the times and dosages of delivery of the medication.

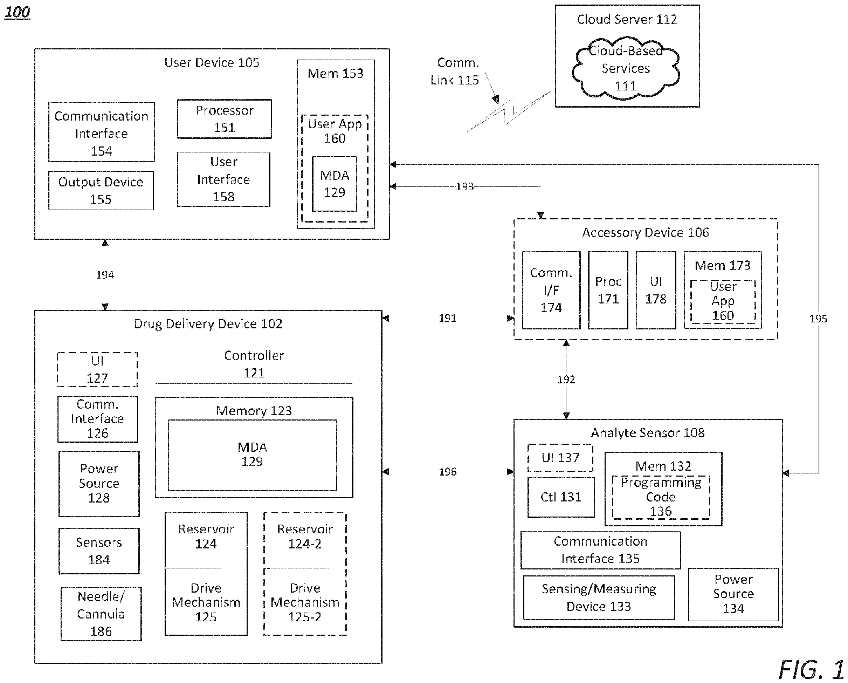

**FIG. 1** illustrates a functional block diagram of an exemplary drug delivery system 100 suitable for implementing the systems and methods described herein. The drug delivery system 100 may implement (and/or provide functionality for) a medication delivery algorithm, such as an artificial pancreas (AP) application, to govern or control the automated delivery of a drug or medication, such as insulin, to a user (e.g., to maintain euglycemia - a normal level of glucose in the blood). The drug delivery system 100 may be an automated drug delivery system that may include a drug delivery device 102 (which may be wearable or attached directly to the skin of a user), an analyte sensor 108 (which may also be wearable), and a user device 105.

Drug delivery system 100, in an optional example, may also include an accessory device 106, such as a smartwatch, a personal assistant device, a smart insulin pen, or the like, which may communicate with the other components of system 100 via either a wired or wireless communication links 191-193.

### *User Device*

The user device 105 may be a computing device such as a smartphone, a smartwatch, a tablet, a personal diabetes management (PDM) device, a dedicated diabetes therapy management device, or the like. In an example, user device 105 may include a processor 151, device memory 153, a user interface 158, and a communication interface 154. The user device 105 may also contain analog and/or digital circuitry that may be implemented as a processor 151 for executing processes based on programming code stored in device memory 153, such as user application 160 incorporating medication delivery algorithm (MDA) 129 to manage a user's blood glucose levels and for controlling the delivery of the drug, medication, or therapeutic agent to the user, as well for providing other functions, such as calculating a carbohydrate-compensation dosage, a correction bolus dosage and the like as discussed below. The user device 105 may be used to activate, deactivate, trigger a needle/canula insertion, program, adjust settings, and/or control operation of drug delivery device 102 and/or the analyte sensor 103 as well as the optional smart accessory device 106.

The processor 151 may also be configured to execute programming code stored in device memory 153, such as the user app 160. The user app 160 may be a computer application that is operable to deliver a drug based on information received from the analyte sensor 103, the cloud-based services 111 and/or the user device 105 or optional accessory device 106. The memory 153 may also store programming code to, for example, operate the user interface 158 (e.g., a touchscreen device, a camera or the like), the communication interface 154 and the like. The processor 151, when executing user app 160, may be configured to implement indications and notifications related to meal ingestion, blood glucose measurements, and the like. The user interface 158 may be under the control of the processor 151 and be configured to present a graphical user interface that enables the input of a meal announcement, adjust setting selections and the like as described herein.

In a specific example, when the user app 160 includes MDA 129, the processor 151 is also configured to execute a diabetes treatment plan (which may be stored in a memory) that is managed by user app 160. In addition to the functions mentioned above, when user app 160 is an AP application, it may further provide functionality to determine a carbohydrate-compensation dosage, a correction bolus dosage and determine a real-time basal dosage according to a diabetes treatment plan. In addition, as MDA 129, user app 160 provides functionality to output signals to the drug delivery device 102 via communications interface 154 to deliver the determined bolus and/or basal dosages.

The communication interface 154 may include one or more transceivers that operate according to one or more radio-frequency protocols. In one embodiment, the transceivers may comprise a cellular transceiver and a Bluetooth® transceiver. The communication interface 154 may be configured to receive and transmit signals containing information usable by user app 160.

User device 105 may be further provided with one or more output devices 155 which may be, for example, a speaker or a vibration transducer, to provide various signals to the user.

### Drug Delivery Device

In various exemplary embodiments, drug delivery device 102 may include a reservoir 124 and drive mechanism 125, which are controllable by controller 121, executing a medication delivery algorithm (MDA) 129 stored in memory 123, which may perform some or all of the functions of the AP application described above, such that user device 105 may be unnecessary for drug delivery device 102 to carry out drug delivery and control. MDA 129 excluding on drug delivery device 102 may be identical to MDA 129 executing on user device 105, but without the user interface screens provided by user app 160. Alternatively, the functionality of MDA 129 may be split between user device 105 and drug delivery device 102.

Controller 121 may act to control reservoir 124 and drive mechanism 125 based on signals received from user app 160 executing on a user device 105 and communicated to drug delivery device 102 via communication link 194. Drive mechanism 125 may operate to longitudinally translate a plunger through the reservoir, so as to force the liquid drug through an outlet fluid port to needle / cannula 186. Alternatively, other types of drive mechanisms may be used.

In an alternate embodiment, drug delivery device 102 may also include an optional second reservoir 124-2 and second drive mechanism 125-2 which enables the independent delivery of two different liquid drugs. As an example, reservoir 124 may be filled with insulin, while reservoir 124-2 may be filled with glucagon, or pramlintide, or GLP-1, for example. In some embodiments, each of reservoirs 124, 124-2 may be configured with a separate drive mechanism 125, 125-2, respectively, which may be separately controllable by controller 121 under the direction of MDA 129. Both reservoirs 124, 124-2 may be connected to a common needle / cannula 186.

Drug delivery device 102 may be optionally configured with a user interface 127 providing a means for receiving input from the user and a means for outputting information to the user. User interface 127 may include, for example, light-emitting diodes, buttons on a housing of drug delivery device 102, a sound transducer, a micro-display, a microphone, an accelerometer for detecting motions of the device or user gestures (e.g., tapping on a housing of the device) or any other type of interface device that is configured to allow a user to enter information and/or allow drug delivery device 102 to output information for presentation to the user (e.g., alarm signals or the like).

Drug delivery device 102 includes a patient interface 186 for interfacing with the user to deliver the liquid drug. Patient interface may be, for example, a needle or cannula for delivering the drug into the body of the user (which may be done subcutaneously, intraperitoneally, or intravenously). Drug delivery device 102 may further include a mechanism for inserting the needle / cannula 186 into the body of the user, which may be integral with or attachable to drug delivery device 102. The insertion mechanism may comprise, in one embodiment, an actuator that inserts the needle / cannula 186 under the skin of the user and thereafter retracts the needle, leaving the cannula in place. The actuator may be triggered by user device 105 or may be a manual firing mechanism comprising springs or other energy storing mechanism, that causes the needle / cannula 186 to penetrate the skin of the user.

In one embodiment, drug delivery device 102 includes a communication interface 126, which may be a transceiver that operates according to one or more radio-frequency protocols, such as Bluetooth®, Wi-Fi, near-field communication, cellular, or the like. The controller 121 may, for example, communicate with user device 105 and an analyte sensor 108 via the communication interface 126.

In some embodiments, drug delivery device 102 may be provided with one or more sensors 184. The sensors 184 may include one or more of a pressure sensor, a power sensor, or the like that are communicatively coupled to the controller 121 and provide various signals. For example, a pressure sensor may be configured to provide an indication of the fluid pressure detected in a fluid pathway between the patient interface 186 and reservoir 124. The pressure sensor may be coupled to or integral with the actuator for inserting the patient interface 186 into the user. In an example, the controller 121 may be operable to determine a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount. In one embodiment, analyte sensor 108 may be integral with drug delivery device 102.

Drug delivery device 102 further includes a power source 128, such as a battery, a piezoelectric device, an energy harvesting device, or the like, for supplying electrical power to controller 121, memory 123, drive mechanisms 125 and/or other components of drug delivery device 102.

Drug delivery device 102 may be configured to perform and execute processes required to deliver doses of the medication to the user without input from the user device 105 or the optional accessory device 106. As explained in more detail, MDA 129 may be operable, for example, to determine an amount of insulin to be delivered, IOB, insulin remaining, and the like and to cause controller 121 to activate drive mechanism 125 to deliver the medication from reservoir 124. MDA 129 may take as input data received from the analyte sensor 108 or from user app 160.

The reservoirs 124, 124-2 may be configured to store drugs, medications or therapeutic agents suitable for automated delivery, such as insulin, Pramlintide, GLP-1, co-formulations of insulin and GLP-1, glucagon, morphine, blood pressure medicines, arthritis drugs, chemotherapy drugs, fertility drugs, hormonal drugs, or the like.

Drug delivery device 102 may be a wearable device and may be attached to the body of a user, such as a patient or diabetic, at an attachment location and may deliver any therapeutic agent mentioned above, to a user at or around the attachment location. A surface of drug delivery device 102 may include an adhesive to facilitate attachment to the skin of a user.

When configured to communicate with an external device, such as the user device 105 or the analyte sensor 108, drug delivery device 102 may receive signals over the wired or wireless link 194 from the user device 105 or from the analyte sensor 108. The controller 121 of drug delivery device 102 may receive and process the signals from the respective external devices as well as implementing delivery of a drug to the user according to a diabetes treatment plan or other drug delivery regimen.

*Accessory Device*

Optional accessory device 106 may be, a wearable smart device, for example, a smart watch (e.g., an Apple Watch®), smart eyeglasses, smart jewelry, a global positioning system-enabled wearable, a wearable fitness device, smart clothing, or the like. Accessory device 106 may alternatively be a smart insulin pen that works with drug delivery device 102 in managing blood glucose and treating diabetes of a user. Similar to user device 105, the accessory device 106 may also be configured to perform various functions including controlling or communicating with drug delivery device 102. For example, the accessory device 106 may include a communication interface 174, a processor 171, a user interface 178 and a memory 173. The user interface 178 may be a graphical user interface presented on a touchscreen display of the smart accessory device 107. The memory 173 may store programming code to operate different functions of the smart accessory device 107 as well as an instance of the user app 160, or a pared-down version of user app 160 with reduced functionality. In some instances, accessory device 107 may also include sensors of various types.

*Analyte Sensor*

The analyte sensor 108 may include a controller 131, a memory 132, a sensing/measuring device 133, an optional user interface 137, a power source/energy harvesting circuitry 134, and a communication interface 135. The analyte sensor 108 may be communicatively coupled to the processor 151 of the management device 105 or controller 121 of drug delivery device 102. The memory 132 may be configured to store information and programming code 136.

The analyte sensor 108 may be configured to detect one or multiple different analytes, such as glucose, lactate, ketones, uric acid, sodium, potassium, alcohol levels or the like, and output results of the detections, such as measurement values or the like. The analyte sensor 108 may, in an exemplary embodiment, be configured as a continuous glucose monitor (CGM) to measure a blood glucose values at a predetermined time interval, such as every 5 minutes, every 1 minute, or the like. The communication interface 135 of analyte sensor 108 may have circuitry that operates as a transceiver for communicating the measured blood glucose values to the user device 105 over a wireless link 195 or with drug delivery device 102 over the wireless communication link 108. While referred to herein as an analyte sensor 108, the sensing/measuring device 133 of the analyte sensor 108 may include one or more additional sensing elements, such as a glucose measurement element, a heart rate monitor, a pressure sensor, or the like. The controller 131 may include discrete, specialized logic and/or components, an application-specific integrated circuit, a microcontroller or processor that executes software instructions, firmware, programming instructions stored in memory (such as memory 132), or any combination thereof.

Similar to the controller 121 of drug delivery device 102, the controller 131 of the analyte sensor 108 may be operable to perform many functions. For example, the controller 131 may be configured by programming code 136 to manage the collection and analysis of data detected by the sensing and measuring device 133.

Although the analyte sensor 108 is depicted in **FIG. 1** as separate from drug delivery device 102, in various embodiments, the analyte sensor 108 and drug delivery device 102 may be incorporated into the same unit. That is, in various examples, the analyte sensor 108 may be a part of and integral with drug delivery device 102 and contained within the same housing as drug delivery device 102 or an attachable housing thereto. In such an example configuration, the controller 121 may be able to implement the functions required for the proper delivery of the medication alone without any external inputs from user device 105, the cloud-based services 111, another sensor (not shown), the optional accessory device 106, or the like.

*Cloud-Based Services*

Drug delivery system 100 may communicate with or receive services from a cloud server 122 providing cloud-based services 111. Services provided by cloud server 112 may include data storage that stores personal or anonymized data, such as blood glucose measurement values, historical IOB or TDI, prior carbohydrate-compensation dosage, and other forms of data. In addition, the cloud-based services 111 may process anonymized data from multiple users to provide generalized information related to TDI, insulin sensitivity, IOB and the like. The communication link 115 that couples the cloud server 112 to other components of system 100, for example, devices 102, 105, 106, 108 of system 100 may be a cellular link, a Wi-Fi link, a Bluetooth® link, or a combination thereof.

*Communication Links*

The wireless communication links 115 and 191-196 may be any type of wireless link operating using known wireless communication standards or proprietary standards. As an example, the wireless communication links 191-196 may provide communication links based on Bluetooth®, Zigbee®, Wi-Fi, a near-field communication standard, a cellular standard, or any other wireless protocol via the respective communication interfaces 126, 135, 154 and 174.

*Operational Example*

In an operational example, user application 160 implements a graphical user interface that is the primary interface with the user and may be used to activate drug delivery device 102, trigger a needle/cannula insertion, start and stop drug delivery device 102, program basal and bolus calculator settings for manual mode as well as program settings specific for automated mode (hybrid closed-loop or closed-loop).

User app 160, provides a graphical user interface 158 that allows for the use of large text, graphics, and on-screen instructions to prompt the user through the set-up processes and the use of system 100. It may also be used to program the user's custom basal insulin delivery profile, accept a recommended basal insulin delivery profile, check the status of drug delivery device 102, initiate bolus doses of insulin, make changes to a patient's insulin delivery profile, handle system alerts and alarms, or allow the user to switch between automated mode and manual mode.

User app 160 may be configured to operate in a manual mode in which user app 160 will deliver insulin at programmed basal rates and user-defined bolus amounts with the option to set temporary basal profiles. The controller 121 will also have the ability to function as a sensor-augmented pump in manual mode, using sensor glucose data provided by the analyte sensor 108 to populate the bolus calculator.

User app 160 may be configured to operate in an automated mode in which user app 160 supports the use of one or multiple target blood glucose values that may be adjusted manually or automatically by the system. For example, in one embodiment, target blood glucose values can range from 110-150 mg/dL, in 10 mg/dL increments, in 5 mg/dL increments, or other increments, but preferably 10 mg/dL increments. The experience for the user will reflect current setup flows whereby the healthcare provider assists the user to program basal rates, glucose targets and bolus calculator settings. These in turn will inform the user app 160 for insulin dosing parameters. The insulin dosing parameters will be adapted over time based on the total daily insulin (TDI) delivered during each use of drug delivery device 102. A temporary hypoglycemia protection mode may be implemented by the user for various time durations in automated mode. With hypoglycemia protection mode, the algorithm reduces insulin delivery and is intended for use over temporary durations when insulin sensitivity is expected to be higher, such as during exercise or fasting.

The user app 160 (or MDA 129) may provide periodic insulin micro-boluses based upon past glucose measurements and/or a predicted glucose over a prediction horizon (e.g., 60 minutes). Optimal post-prandial control may require the user to give meal boluses in the same manner as current pump therapy, but normal operation of the user app 160 will compensate for missed meal boluses and mitigate prolonged hyperglycemia. The user app 160 uses a control-to-target strategy that attempts to achieve and maintain a set target glucose value, thereby reducing the duration of prolonged hyperglycemia and hypoglycemia.

In some embodiments, user device 105 and the analyte sensor 108 may not communicate directly with one another. Instead, data (e.g., blood glucose readings) from analyte sensor may be communicated to drug delivery device 102 via link 196 and then relayed to user device 105 via link 194. In some embodiments, to enable communication between analyte sensor 108 and user device 105, the serial number of the analyte sensor must be entered into user app 160.

User app 160 may provide the ability to calculate a suggested bolus dose through the use of a bolus calculator. The bolus calculator is provided as a convenience to the user to aid in determining the suggested bolus dose based on ingested carbohydrates, most-recent blood glucose readings (or a blood glucose reading if using fingerstick), programmable correction factor, insulin to carbohydrate ratio, target glucose value, and insulin on board (IOB). IOB is estimated by user app 160 taking into account any manual bolus and insulin delivered by the algorithm.

*Description of Embodiments*

In a first aspect of the invention, an evaluation is undertaken to determine the user's risk of experiencing a hyperglycemic or hypoglycemic condition. In preferred embodiments of the invention, to increase confidence in the assessment, the determination is made based on several factors. The factors may then be combined to produce a combined assessment of the risk of either hyperglycemia or hypoglycemia.

In some embodiments, the evaluation of the risk may be undertaken automatically by ADD system 100. In certain embodiments, MDA 129 may periodically receive new readings from CGM 108, for example, once per 5-minute cycle. Each time a new CGM reading is received, MDA 129 may undertake a variety of calculations, including the determination of the risk of hypoglycemia or hypoglycemia in accordance with embodiments disclosed herein. In other embodiments, the risk assessment may be performed at the request of the user. The user may request the assessment, for example, by using user interface 158 of user device 105 to interface with the user app 160.

In one embodiment, the assessment of risk for both hyperglycemia and hypoglycemia may be based on four factors: the current CGM value, the CGM trend, the insulin-on-board (IOB) and a factor indicating the accuracy of previous risk assessments. These four factors are combined into a final determination of the risk. Specific methods of evaluating each of the four factors, as well as a method of combining the factors, are provided in detail below. In other embodiments, other factors, as well as other methods of combining the factors, are contemplated to be within the scope of the invention.

In an embodiment of the invention described in detail herein, the factors that may be used for determining the hyperglycemic and hypoglycemic risks may be expressed as probabilities.

For the hypoglycemic assessment, in one embodiment, a (first) risk factor based on the CGM value factor may be determined as a linear increase in the probability of hypoglycemia as the user's CGM reading decreases from a first threshold blood glucose value (for the first risk factor),, e.g. 150 mg/dL to a second threshold blood glucose value (for the first risk factor),, e.g. 70 mg/dL, with 100% probability when the CGM reading value is the second threshold blood glucose value, e.g. 70 mg/dL. In some embodiments, the first risk factor based on the CGM value factor is determined by assessing the negative deviation of the CGM reading value from the first threshold blood glucose value. The first threshold blood glucose value may be the target blood glucose value, or the lower value of a target blood glucose value range. In some embodiments, the first risk factor based on the CGM value factor is determined by assessing the positive deviation of the CGM reading value from the second threshold blood glucose value, in particular a hypoglycemia threshold value. The hypoglycemia threshold value may be the numerical value at which the user is considered as being hypoglycemic. In some embodiments, the first risk factor based on the CGM value factor is determined by assessing where the CGM reading value is between the second threshold blood glucose value and the first threshold blood glucose value, wherein the first risk factor is determined to be 0% when the CGM reading value is at or above the first threshold blood glucose value, the first risk factor is determined to be 100% when the CGM reading value is at or below the second threshold blood glucose value, and wherein CGM reading values between the first threshold blood glucose value and the second threshold blood glucose value have a risk between 0% to 100%. In some embodiments, the CGM reading values between the first threshold blood glucose value and the second threshold blood glucose value are attributed a risk between 0% to 100% based on a linear increase the closer the CGM reading values are to the second threshold blood glucose value. For example, for a first threshold blood glucose value at 150 mg/dL and a hypoglycemia threshold at 70 mg/dL, every negative deviation of 8 mg/dL from the first threshold blood glucose value may result in the first risk factor increasing by 10%, e.g. 134 mg/dL would result in the first risk factor being 20%. In some embodiments, the correlation between the first risk factor, the first threshold blood glucose value and the second threshold blood glucose value may be non-linear. Other blood glucose values can be used than the thresholds mentioned above. Using the exemplary values mentioned above, the probability can be expressed as follows:

$$P(i)_{CGM\ Value} = min\left(1, max\left(0, \frac{150 - CGM(i)}{80}\right)\right)$$

(1)

where:

$CGM(i)$ is the value of the CGM reading received during the current cycle of MDA 129.

Accordingly, in some embodiments, the first risk factor based on the CGM value factor is determined by the following steps:

calculating a first value(for the first risk factor) as a first threshold blood glucose value subtracted by the value of the CGM reading received during the current cycle of MDA 129, and
calculating a second value(for the first risk factor) by dividing the first value by a third threshold blood glucose value,
determining a third value (for the first risk factor),, wherein the third value is set to 0 if the second value is smaller than 0, and wherein the third value is set to the second value if the second value is greater than 0,
determining the first risk factor based on the CGM value factor to be 1 if the third value is greater than 1, and
determining the first risk factor to be the third value if the third value is smaller than 1.

In some embodiments, the first threshold blood glucose value is preset by the user or a healthcare provider. In some embodiments, the first threshold blood glucose value is between about 120 mg/dL to about 200 mg/dL, more specifically between about 130 mg/dL to about 180 mg/dL, and in particular between about 140 mg/dL to about 160 mg/dL. In some embodiments, the third threshold blood glucose value is preset by the user or healthcare provider. In some embodiments, the third threshold blood glucose value is between about 50 mg/dL to about 130 mg/dL, more specifically between about 60 mg/dL to about 110 mg/dL, and in particular between about 70 mg/dL to about 90 mg/dL. In some embodiments, the third threshold blood glucose value is calculated as the first threshold blood glucose value subtracted by a hypoglycemia threshold value.

As can be seen, in Eq. (1), as the CGM reading for the current cycle approaches 70 mg/dL, the fraction in Eq. (1) becomes equal to 1, yielding a 100% probability of hypoglycemia for this factor. In other embodiments, other thresholds may be used. Specifically, thresholds specified by the constants in the equations specified herein may be modified, for example on an individual basis.

A second factor (second risk factor) that may be used in this embodiment in determining the hypoglycemic risk assessment is the CGM trend, which may be determined as a linear increase in the probability of hypoglycemia as the rate of change of the user's blood glucose level in a previous timeframe, e.g. in the previous 15 minutes (i.e., 3 cycles in the exemplary ADD system 100 described herein using 5-minute cycles), gets closer to a first rate of change, e.g.-60 mg/dL, or a decrease of 20 mg/dL/cycle, with 100% probability of hypoglycemia indicated when the rate of change is the first rate of change, e.g. -20 mg/dL/cycle or worse. In some embodiments, the second risk factor based on the CGM trend is determined by assessing the negative rate of change of the CGM reading value from a first threshold blood glucose value (for the second risk factor) towards a second threshold blood glucose value (for the second risk factor)..
The first threshold blood glucose value may be the target blood glucose value, or the lower value of a target blood glucose value range. In some embodiments, the second risk factor is determined by assessing the rate of change of the CGM reading value towards a hypoglycemia threshold value. The hypoglycemia threshold value may be the numerical value at which the user is considered as being hypoglycemic. In some embodiments, the second risk factor is determined by assessing what rate of change the CGM reading value has relative to a negative rate of change threshold value, wherein the second risk factor is determined to be 0% when the CGM reading value rate of change is 0 or above and the second risk factor is determined to be 100% when the CGM reading value is at or below the negative rate of change threshold value, and wherein CGM reading value rates of change between 0 and the negative rate of change threshold have a risk between 0% to 100%. In some embodiments, the CGM reading rates of change between 0 and the negative rate of change threshold are attributed a risk between 0% to 100% based on a linear increase the closer the CGM reading value rate of change is to the negative rate of change threshold value. In some embodiments, the correlation between the second risk factor and the CGM reading value rate of change may be non-linear. Other values than those mentioned above may be used. Using these exemplary values mentioned above, the probability can be expressed by Eq. (2):

$$P(i)_{CGM\ Trend} = min\left(1, max\left(0, -\frac{CGM(i) - CGM(i-3)}{60}\right)\right)$$

(2)

where:

CGM(*i*) is the value of the CGM reading received during the current cycle of MDA 129. Eq. (2) assumes that ADD system 100 is using a 5-minute cycle and that the risk is dependent on a trend over the last 3 cycles; however, other cycle durations and numbers of cycles may be used. Accordingly, in some embodiments, the second risk factor is determined by the following steps: calculating a first value (for the second risk factor) as the negative value of the CGM reading received during the current cycle of MDA 129 subtracted by a CGM reading received during a previous cycle of MDA 129, and

calculating a second value(for the second risk factor) by dividing the first value by a second blood glucose value, determining a third value(for the second risk factor), wherein the third value is set to 0 if the second value is smaller than 0, and wherein the third value is set to the second value if the second value is greater than 0, determining the second risk factor to be 1 if the third value is greater than 1, and determining the second risk factor to be the third value if the third value is smaller than 1.

In some embodiments, the CGM reading received during a previous cycle of MDA 129 is a the CGM reading received 1 to 6 cycles prior to the current cycle of the MDA 129, more specifically 2 to 5 cycles prior to the current cycle and in particular between about 3 to 4 cycles prior to the current cycle. In some embodiments, the second threshold blood glucose value is calculated by multiplying a rate of change threshold value with the number of cycles the CGM reading received during a previous cycle of MDA 129 is prior to the current cycle. For example, if the CGM reading received during a previous cycle of MDA 129 has been determined 3 cycles prior to the current cycle, the rate of change threshold value is multiplied by 3. In some embodiments, the rate of change threshold value is set by the user or a healthcare provider. In some embodiments, the rate of change threshold value is between about 5 mg/dL/cycle to about 40 mg/dL/cycle, more specifically between about 10 mg/dL/cycle to about 30 mg/dL/cycle, and in particular between about 15 mg/dL/cycle to about 25 mg/dL/cycle.

A third factor (third risk factor) that may be used in this embodiment in determining the hypoglycemic risk assessment is the insulin-on-board (IOB), wherein the insulin-on-board is the insulin remaining in the user's body. Eq. (3a) estimates the insulin-on-board necessary to bring the user's blood glucose to zero, with the consideration that it compensates a possible meal equivalent to 150 mg/dL, plus a correction, to bring the user's current blood glucose to 150 mg/dL. The net result is a maximum insulin-on-board necessary to bring the current blood glucose to 0 mg/dL. The probability ranges from 0% for no insulin-on-board to 100% for insulin-on-board exceeding what is necessary to correct to a blood glucose of 0 mg/dL. In some embodiments, the third risk factor is based on a current insulin-on-board, more specifically the third risk factor increases with increasing insulin-on-board, in particular relative to the current blood glucose reading of the user. The third risk factor may increase linearly with increasing insulin-on-board. Eq. (3a) estimates a third risk factor based on excess insulin-on-board.:

$$P(i)_{IOB} = max\left(0, min\left(1, \frac{CF(i) * IOB(i)}{CGM(i)}\right)\right)$$

(3a)

where:

CF(*i*) is the user's correction factor for cycle *i*;
IOB(*i*) is the insulin-on-board for cycle *i*; and
CGM(*i*) is the current blood glucose reading for cycle *i*.

Accordingly, in some embodiments, the third risk factor based on the insulin-on-board is determined by the following steps:

calculating a first value (for the third risk factor) by multiplying the user's correction factor for cycle *i* with the insulin-on-board for cycle *i*, and

calculating a second value (for the third risk factor) by dividing the first value by the current blood glucose reading for cycle *i* (of MDA 129),

determining a third value (for the third risk factor), wherein the third value is set to 1 if the second value is greater than 1, and wherein the third value is set to the second value if the second value is smaller than 1,

determining the third risk factor based on the insulin-on-board to be 0 if the third value is smaller than 0, and determining the third risk factor to be the third value if the third value is greater than 0. Alternatively, the third factor may be estimated as specified in Eq. (3b), which calculates a linear increase in the probability of hypoglycemia as a conversion of the excess *IOB* beyond the quantity required for the user to get to a blood glucose value below 150 mg/dL, with a 100% probability of hypoglycemia if the resulting blood glucose concentration can lower the user's blood glucose level to below 0 mg/dL. This takes into account that the typical meal bolus can account for approximately 40g of carbohydrates, which corresponds to an approximate 144 mg/dL increase in blood glucose (approximately 3.6 mg/dL increase in blood glucose per 1g of carbohydrates). Therefore, if excess *IOB* is beyond this amount, this factor predicts a 100% chance of hypoglycemia. This probability can be expressed as follows:

$$P(i)_{IOB} = max\left(0, min\left(1, \frac{CF(i) * IOB(i)}{min(CGM(i), 150)}\right)\right)$$

(3b)

Accordingly, in some embodiments, third the risk factor based on the insulin-on-board is determined by the following steps:

calculating a first value (for the third risk factor) by multiplying the user's correction factor for cycle *i* with the insulin-on-board for cycle *i*, and

calculating a second value (for the third risk factor) by dividing the first value by the smaller value of the current blood glucose reading for cycle *i* (of MDA 129) or a meal accounting value, determining a third value (for the third risk factor), wherein the third value is set to 1 if the second value is greater than 1, and wherein the third value is set to the second value if the second value is smaller than 1,

determining the third risk factor based on the insulin-on-board to be 0 if the third value is smaller than 0, and determining the third risk factor to be the third value if the third value is greater than 0. In some embodiments, the meal accounting value for calculating the third risk factor accounts for a rise in the blood glucose value than can be achieved by consuming a meal. In some embodiments, the meal accounting value is preset by the user or a healthcare provider. In some embodiments, the meal accounting value is between about 120 mg/dL to about 200 mg/dL, more specifically between about 130 mg/dL to about 180 mg/dL, and in particular between about 140 mg/dL to about 160 mg/dL.

A fourth factor that may be used for assessing the risk of hypoglycemia includes a personalization factor which determines if the user's glucose concentration for a time period, e.g. for up to 30 minutes, after a number of previous determinations of hypoglycemic risk (resulting in an alert to the user) was below a hypoglycemic threshold value, e.g. 100 mg/dL, which implies that previous assessments of a risk existed for hypoglycemia were accurate. Other values may be used. In one embodiment, the accuracy is determined over the last 3 alerts and for a 30-minute period after the alerts, but other numbers of alerts and time periods may be used. In some embodiments, the accuracy is determined over the last 2 to 20, more specifically, 3 to 10 alerts. In some embodiments, the accuracy is determined for a 5 minute to 120 minute, more specifically a 10 minute to 60 minute and in particular for 20 minute to 45 minute period after the alters. Note that this factor is not evaluated as a probability but instead is evaluated as a binary value (0% or 100%). Eq. (4) makes this determination for each of the $k^{th}$ previous alerts:

$$P(i)_{Personal} = \begin{cases} 1 & G(A_{k-1} + 1 \ldots 6) < 100, G(A_{k-2} + 1 \ldots 6) < 100, G(A_{k-3} + 1 \ldots 6) < 100 \\ 0 & otherwise \end{cases}$$

(4)

where:
$G(A_{k-1\ldots 3} + 1\ldots 6)$ represents blood glucose readings for a 30-minute time period beginning at the cycle when the last alert was raised. Note that this assumes MDA 129 operates on 5-minutes cycles such that "1 ... 6" represents 30 minutes.

MDAs using cycles of other time durations would necessarily change these constants.

Note that in certain embodiments, the value of this factor may be set to "1" (100%) when the user's blood glucose level fell below 100 mg/dL after *each* of the last three alerts (AND condition), or if the blood glucose level fell below 100 mg/dL after *any* of the last three alerts (OR condition).

In certain embodiments, a hypoglycemia alert would be triggered if at least three of the four factors previously discussed show a risk of hypoglycemia but will not be triggered otherwise. Therefore, in this example, if the previous three alerts did not result in a blood glucose concentration below 100 mg/dL (for example, as assessed by the fourth factor), the user would have to meet all three real-time indicators to the maximum probability of "1" to be provided with an alert. If the previous three alerts did result in a blood glucose concentration below 100 mg/dL, then the combination of the three real time indicators only need to exceed a sum of "2" to generate this alert. Therefore, a probability-alert threshold may be established, as follows:

$$A(i)_{k,hypo} = \begin{cases} 1 & P_{CGM\ Value} + P_{CGM\ Trend} + P_{IOB} + P_{Personal} \geq 3 \\ 0 & otherwise \end{cases}$$

$$(5)$$

where:

$A(i)_{k,hypo}$ indicates whether or not an alert should be raised (i.e., $A(i)_{k,hypo}$ = 1) during cycle *i*.

As would be realized by one of skill in the art, the constants in Eqs. (1-4) may, in some embodiments, be changed based on a large population of users, and in other embodiments, may be personalized for individual users or changes to work with ADD systems other than ADD system 100 described herein. In some embodiments, MDA 129 may initially use the constants specified in Eqs. (1-4) or other constants based on a large population of users and may thereafter adjust the constants for specific individual users. As will be further realized by one of skill in the art, any number of factors could be evaluated to increase or decrease the certainty of the risk assessment and the necessity to trigger an alert. In yet other embodiments, the individual terms indicating each real time indicator in Eq. (5) may be fractionally varied with differing coefficients to increase or decrease the sensitivity of the occurrence of an alert.

A similar methodology may be used to determine the risk of hyperglycemia and the same or similar factors may be used. For the hyperglycemic assessment, the CGM value factor (fifth risk factor), as evaluated by Eq. (6), may be determined as a linear increase in the probability of hyperglycemia as the CGM readings increase from 180 mg/dL to 300 mg/dL, with 100% probability when the user's blood glucose level is 300 mg/dL.. In some embodiments, the fifth risk factor based on the CGM value factor is determined by assessing the positive deviation of the CGM reading value from a first threshold blood glucose value (for the fifth risk factor). The first threshold blood glucose value may be the target blood glucose value, or the upper value of a target blood glucose value range. In some embodiments, the fifth risk factor based on the CGM value factor is determined by assessing the positive deviation of the CGM reading value from a hyperglycemia threshold value. The hyperglycemia threshold value may be the numerical value at which the user is considered as being hyperglycemic. In some embodiments, the fifth risk factor based on the CGM value factor is determined by assessing where the CGM reading value is between the hyperglycemia threshold value and the first threshold blood glucose value, wherein the risk is considered to be 0% when the CGM reading value is at or below the first threshold blood glucose value, the risk is considered to be 100% when the CGM reading value is at or above the hyperglycemia threshold value, and wherein CGM reading values between the first threshold blood glucose value and the hypoglycemia threshold have a risk factor between 0% to 100%. In some embodiments, the CGM reading values between the first threshold blood glucose value and the hyperglycemia threshold are attributed a risk between 0% to 100% based on a linear increase the closer the CGM reading values are to the hyperglycemia threshold value. For example, for a first threshold blood glucose value at 180 mg/dL and a hypoglycemia threshold at 300 mg/dL, every positive deviation of 12 mg/dL from the first threshold blood glucose value may result in the fifth risk factor increasing by 10%, e.g. 204 mg/dL would result in the fifth risk factor being 20%. In some embodiments, the correlation between the fifth risk factor, the first threshold blood glucose value and the hyperglycemia threshold value may be non-linear. Other blood glucose values may be used than the threshold mentioned above. Using these exemplary values mentioned above, the probability can be expressed as follows:

$$P(i)_{CGM\ Value} = min\left(1, max\left(0, \frac{CGM(i) - 180}{120}\right)\right)$$

$$(6)$$

where:

*CGM(i)* is the value of the CGM reading received during the current cycle of MDA 129.
Accordingly, in some embodiments, the fifth risk factor based on the CGM value factor is determined by the following steps:

    calculating a first value (for the fifth risk factor) as the CGM reading received during the current cycle of MDA 129 subtracted by the value of a first threshold blood glucose value(for the fifth risk factor), and
    calculating a second value(for the fifth risk factor) by dividing the first value by a second threshold blood glucose value (for the fifth risk factor),
    determining a third value (for the fifth risk factor), wherein the third value is set to 0 if the second value is smaller than 0, and wherein the third value is set to the second value if the second value is greater than 0,
    determining the fifth risk factor based on the CGM value factor to be 1 if the third value is greater than 1, and determining the fifth risk factor to be the third value if the third value is smaller than 1.

In some embodiments, the first threshold blood glucose value is preset by the user or a healthcare provider. In some embodiments, the first threshold blood glucose value is between about 150 mg/dL to about 220 mg/dL, more specifically between about 160 mg/dL to about 200 mg/dL, and in particular between about 170 mg/dL to about 190 mg/dL. In some embodiments, the second threshold blood glucose value is preset by the user or healthcare provider. In some embodiments, the second threshold blood glucose value is between about 80 mg/dL to about 150 mg/dL, more specifically between about 100 mg/dL to about 140 mg/dL, and in particular between about 110 mg/dL to about 130 mg/dL. In some embodiments, the second threshold blood glucose value is calculated as a hyperglycemia threshold value subtracted by the first threshold blood glucose value.

As can be seen, in Eq. (6), as the CGM reading for the current cycle approaches 300 mg/dL, the fraction in Eq. (6) becomes equal to 1, yielding a 100% probability for this factor. In other embodiments, other thresholds may be used. Specifically, thresholds specified by the constants in the equations specified herein may be modified, for example on an individual basis.

Eq. (7) may be used to calculate the CGM trend factor for hyperglycemia (sixth risk factor), which is determined as a linear increase in the probability of hyperglycemia as the rate of change of the user's blood glucose level in a previous timeframe, e.g. the previous 15 minutes (i.e., 3 cycles in the exemplary ADD system 100 described herein using 5-minute cycles), gets closer to a first rate of change, e.g. 60 mg/dL, or an increase of 20 mg/dL/cycle, with 100% probability of hypoglycemia indicated when the rate of change is 20 mg/dL/cycle or worse. In some embodiments, the sixth risk factor based on the CGM trend is determined by assessing the positive rate of change of the CGM reading value from a first threshold blood glucose value (for the sixth risk factor) towards a second threshold blood glucose value (for the sixth risk factor). The first threshold blood glucose value may be the target blood glucose value, or the upper value of a target blood glucose value range. In some embodiments, the sixth risk factor is determined by assessing the rate of change of the CGM reading value towards a hyperglycemia threshold value. The hyperglycemia threshold value may be the numerical value at which the user is considered as being hyperglycemic. In some embodiments, the sixth risk factor is determined by assessing what rate of change the CGM reading value has relative to a positive rate of change threshold value, wherein the sixth risk factor is determined to be 0% when the CGM reading value rate of change is 0 or below and the sixth risk factor is determined to be 100% when the CGM reading value is at or above the negative rate of change threshold value, and wherein CGM reading value rates of change between 0 and the positive rate of change threshold have a risk factor between 0% to 100%. In some embodiments, the CGM reading rates of change between 0 and the positive rate of change threshold are attributed a risk between 0% to 100% based on a linear increase the closer the CGM reading value rate of change is to the positive rate of change threshold value. In some embodiments, the correlation between the sixth risk factor and the CGM reading value rate of change may be non-linear. Other values than those mentioned above may be used. Using these exemplary values mentioned above, the probability can be expressed by Eq. (7):

$$P(i)_{CGM\ Trend} = min\left(1, max\left(0, \frac{CGM(i) - CGM(i-3)}{60}\right)\right)$$

$$(7)$$

where:

*CGM(i)* is the value of the CGM reading received during the current cycle of MDA 129. Eq. (7) assumes that ADD system 100 is using a 5-minute cycle and that the risk is dependent on a trend over the last 3 cycles. However, other cycle durations and numbers of cycles may be used.

Accordingly, in some embodiments, the sixth risk factor is determined by the following steps: calculating a first value (for the sixth risk factor) as the CGM reading received during the current cycle of MDA 129 subtracted by a CGM reading received during a previous cycle of MDA 129, and calculating a second value (for the sixth risk factor) by dividing the first value by a second threshold blood glucose value (for the sixth risk factor),

determining a third value (for the sixth risk factor), wherein the third value is set to 0 if the second value is smaller than 0, and wherein the third value is set to the second value if the second value is greater than 0,
determining the sixth risk factor to be 1 if the third value is greater than 1, and determining the sixth risk factor to be the third value if the third value is smaller than 1.

In some embodiments, the CGM reading received during a previous cycle of MDA 129 is a the CGM reading received 1 to 6 cycles prior to the current cycle of the MDA 129, more specifically 2 to 5 cycles prior to the current cycle and in particular between about 3 to 4 cycles prior to the current cycle.

In some embodiments, the second threshold blood glucose value is calculated by multiplying a rate of change threshold value with the number of cycles the CGM reading received during a previous cycle of MDA 129 is prior to the current cycle. For example, if the CGM reading received during a previous cycle of MDA 129 has been determined 3 cycles prior to the current cycle, the rate of change threshold value is multiplied by 3. In some embodiments, the rate of change threshold value is set by the user or a healthcare provider. In some embodiments, the rate of change threshold value is between about 5 mg/dL to about 40 mg/dL, more specifically between about 10 mg/dL to about 30 mg/dL, and in particular between about 15 mg/dL to about 25 mg/dL.

As with the assessment for hypoglycemia, the insulin-on-board factor (seventh risk factor) may be evaluated using one of two alternate methods. Eq. (8a) estimates the linear increase in the probability of hypoglycemia as the conversion of the current IOB to the user's final blood glucose concentration remains above a first blood glucose threshold value (for the seventh risk factor), e.g. 100 mg/dL, with 100% probability being reached if there is insufficient IOB to keep the user below a second blood glucose threshold value (for the seventh risk factor), e.g. 180 mg/dL. This probability can be expressed as follows:

$$P(i)_{IOB} = \min \; max \left( 0, \frac{\frac{IOB(i)}{CF(i)} - 100}{80} \right)$$

$$(8a)$$

where:

$CF(i)$ is the correction factor for cycle $i$; and
$IOB(i)$ is the insulin-on-board for cycle $i$.

Alternatively, the *IOB* factor may be estimated as specified in Eq. (8b), which is based on the assumption that if the current *IOB* is insufficient to bring the blood glucose level to the second threshold blood glucose value (for the seventh risk factor), e.g. 180 mg/dL, absent a meal, the hyperglycemia risk is 100%. However, if the current *IOB* is sufficient to bring the blood glucose level to the first threshold blood glucose value (for the seventh risk factor), e.g. 100 mg/dL, the risk of hyperglycemia is 0%. Using these values, this probability can be expressed as follows:

$$P(i)_{IOB} = min \left( 1, max \left( 0, \frac{CGM(i) - 100 - CF(i)IOB(i)}{80} \right) \right)$$

$$(8b)$$

Accordingly, in some embodiments, the seventh risk factor based on the insulin-on-board is determined by the following steps:

calculating a first value (for the seventh risk factor) by multiplying the user's correction factor for cycle *i* with the insulin-on-board for cycle *i*, and

calculating a second threshold blood glucose value (for the seventh risk factor) by subtracting from the current blood glucose reading for cycle *i* (of MDA 129) the first value (for the seventh risk factor) and the first threshold blood glucose value (for the seventh risk factor),

calculating a third value (for the seventh risk factor) by subtracting the first threshold blood glucose value from the second threshold blood glucose value,

calculating a fourth value (for the seventh risk factor) by dividing the second threshold blood glucose value by the third value,

determining a fifth value (for the seventh risk factor), wherein the fifth value is set to 0 if the fourth value is smaller than 0, and wherein the fifth value is set to the fourth value if the fourth value is greater than 0,

determining the seventh risk factor based on the insulin-on-board to be 1 if the fifth value is greater than 1, and determining the seventh risk factor to be the fifth value if the fifth value is smaller than 1.

The personalization factor for expressing the risk of hyperglycemia, expressed by Eq. (9), determines if the user's glucose concentration for up to 1 hour after a previous number of alerts (i.e., determinations of hyperglycemic risk resulting in an alert to the user) resulted in the glucose concentration above 240 mg/dL, which implies that previous assessments of a risk existed for hypoglycemia were accurate. In one embodiment, the accuracy is determined over the last 3 alerts and for a 30-minute period after the alerts, but other numbers of alerts and time periods may be used. Note that this factor is not evaluated as a probability but instead is evaluated as a binary value (0% or 100%). Eq. (9) makes this determination for each of the $k^{th}$ previous alerts:

$$P(i)_{Personal} = \begin{cases} 1 & G(A_{k-1} + 1 \dots 12) > 240, G(A_{k-2} + 1 \dots 12) > 240, G(A_{k-3} + 1 \dots 12) > 240 \\ 0 & otherwise \end{cases}$$

$$(9)$$

where:

$G(A_{k-1\dots3} + 1 \dots 12)$ represents blood glucose readings for a 1 hour time period beginning at the cycle when the last alert was raised. Note that this assumes MDA 129 operates on 5-minutes cycles such that "1 ... 12" represents 1 hour. MDAs using cycles of other time durations would necessarily change these constants.

An additional assessment for hyperglycemic risk can be made based on the previous four factors. In certain embodiments, a hyperglycemia alert would be triggered if at least three of the four factors for determining hyperglycemic risk previously discussed show a risk of hyperglycemia but will not be triggered otherwise. Therefore, in this example, if the previous three alerts did not result in the glucose concentration above 240 mg/dL (for example, as assessed by the personalization factor above), the user would have to meet all three real-time indicators to be provided with an alert. Otherwise, if the previous three alerts did result in a blood glucose concentration above 240 mg/dL, then the combination of the three real time indicators only need to exceed a sum of "2" to generate this alert. This is expressed in the following equation:

$$A(i)_{k,hyper} = \begin{cases} 1 & P_{CGM\,Value} + P_{CGM\,Trend} + P_{IOB} + P_{Personal} \geq 3 \\ 0 & otherwise \end{cases}$$

$$(10)$$

where:

$A(i)_{k,hyper}$ indicates whether or not an alert should be raised during cycle *i*.

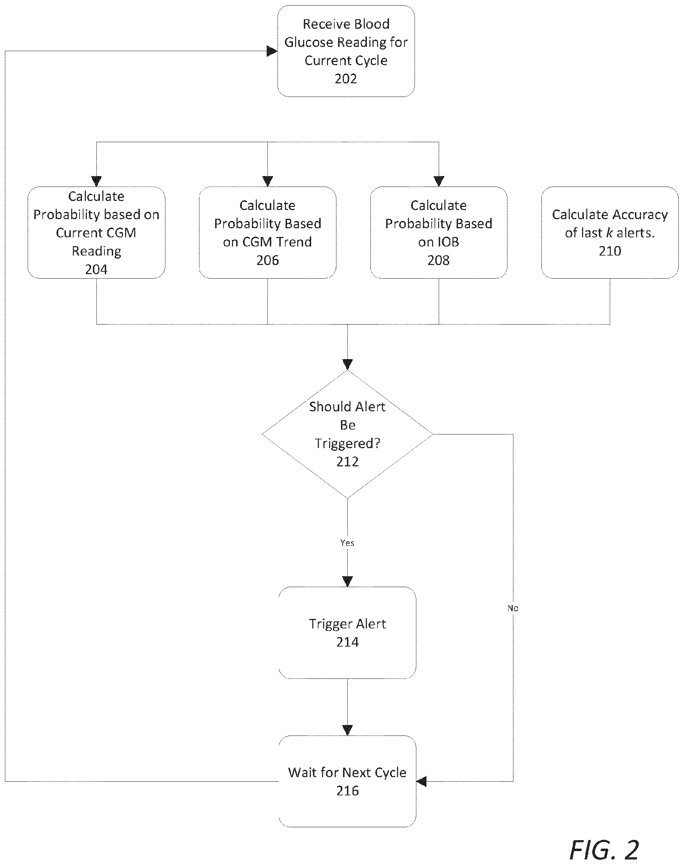

FIG. 2 is a flowchart showing the process for calculating the risk for both hypoglycemia and hyperglycemia during each cycle. This process may be performed by MDA 129 executing on user device 105 or on drug delivery device 102. At step 202, a new blood glucose reading is received from sensor 108. Upon receipt of the new blood glucose reading, the risk factor based on the current CGM reading is calculated at step 204, the risk factor based on the CGM trend is calculated at step 206 and the risk factor based on *IOB* is calculated at step 208. Because the risk factor based on the accuracy of the previous alerts is not based on the current blood glucose reading, this risk factor may be calculated, at step 210, at any time. At decision point 212, it is decided, for example based on the four factors in accordance with Eq. (5) for hypoglycemia or Eq. (10) for hyperglycemia, if an alert should be raised. If it is determined that an alert should be raised, at step 214 the alert is triggered and will be delivered to the user as described below. Thereafter, the process continues to step 216 to await the arrival of the next blood glucose reading at 202. It is intended that the process repeat each cycle. However, in some embodiments, the process may be run at multiple intervals of cycles or only at the user's request.

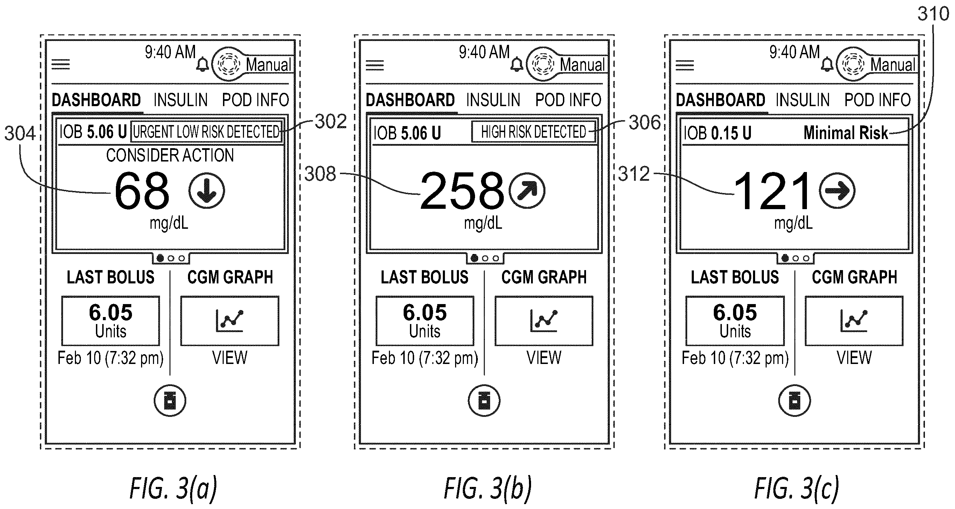

Once the decision is made at 212 to trigger an alert, the alert is triggered at 214. There several methods of alerting the user. In one embodiment, shown in **FIGS. 3(a-c),** a flag is displayed on a default screen of user application 160 executing on user device 105. Several exemplary embodiments are shown in the figures. **FIG. 3(a)** shows the condition where it has been determined that a risk of hypoglycemia exists. As a result, the text flag 302 is shown on the screen (for example, in red text; other colors, text, or graphics may be used), providing a warning to the user of the risk of hypoglycemia, and the current blood glucose reading 304 may be shown in a red color. The arrow next to the blood glucose reading indicates a downward trend of the user's blood glucose readings. **FIG. 3(b)** shows the condition where it has been determined that a risk of hyperglycemia exists. Text flag 306 is shown on the screen (for example, in yellow text; other colors, text, or graphics may be used), providing a warning to the user of the risk of hyperglycemia. In addition, the current blood glucose reading 308 may be shown in yellow to indicate a high reading and the trend arrow next to the blood glucose reading indicates that the user's blood glucose levels are trending upward. If, at decision point 212, it is determined that no alert is required, the screen shown in **FIG. 3(c)** may be displayed showing a text flag indicating a minimal risk at 310 and the user's current blood glucose reading 312, which may be shown in blue to indicate a normal level (other colors, text, or graphics may be used).

In some embodiments, if it is determined that no alert is required, user app 160 may simply do nothing instead of showing the minimal risk flag 310. As would be realized by one of skill in the art, the invention is not to be limited by the actual text and colors of flags 302, 306, 310 and blood glucose readings 304, 308, 312, shown in **FIGS. 3(a-c),** which are shown only as examples of one implementation of this embodiment.

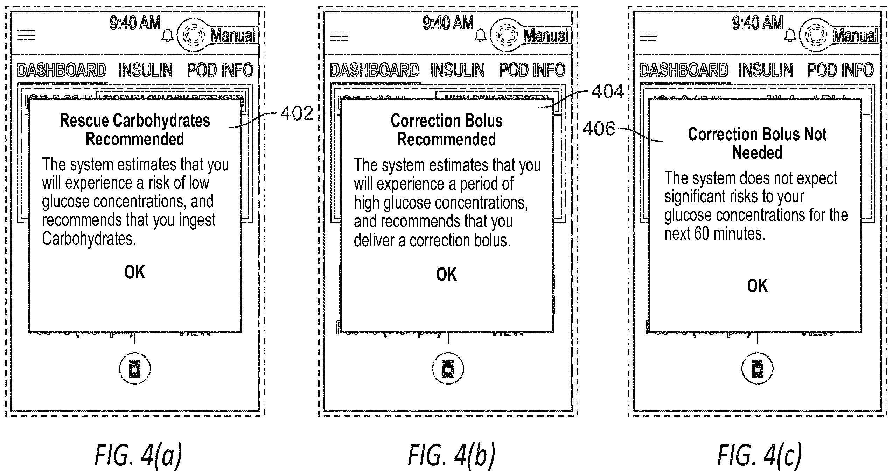

The modal windows shown in **FIGS. 4(a-c)** may be displayed in the user interface of user app 160 in lieu of or in addition to the elements shown in **FIGS. 3(a-c)**. **FIG. 4(a)** shows modal window 402 displayed when a risk of hypoglycemia is determined. The window recommends that the user ingest carbohydrates to raise the blood glucose level. **FIG. 4(b)** shows modal window 404 displayed when the system detects a risk of hyperglycemia. Model window 404 recommends a correction bolus be administered by the user. Alternatively, the user may be asked to confirm that a correction bolus, as determined by the system, may be delivered. **FIG. 4(c)** shows modal window 406 which may be displayed when no risk is detected. Any of the exemplary modal windows 402, 404, 406 shown in **FIGS. 4(a-c)** may be dismissed by the user by pressing the "OK" button in the modal window. Alternatively, exemplary modal windows 402, 404, 406 may be dismissed automatically after a predetermined period of time. As with the user interface elements shown in **FIG. 3(c),** if no risk is detected, the user app 160 may simply display no modal window in lieu of displaying modal window 406. As would be realized by one of skill in the art, the actual text of the modal windows 402, 404, 406 shown in **FIGS. 4(a-c)** are exemplary only and are not meant to limit the scope of the invention.

In some embodiments of the invention, it is possible that user device 105 may not be present, in which case MDA 129 running on drug delivery device 102 would assess the probability of risk for both hypoglycemia and hyperglycemia and would inform the user via user interface 127 which may consist of audio, visual, or haptic interfaces. In such a case, to alert the user, the drug delivery device may use sound, for example a series of beeps, a visual indicator, for example, a series of blinking lights, or some form of haptic feedback, for example, a vibrating element in the drug delivery device 102, to provide a series of vibrations that can be sensed by the user. Any of the foregoing means of alerting the user, or combination thereof, may be used by MDA 129 running on drug delivery device 102. In addition, drug delivery device 102 may provide an indication of the risk and the necessity of raising an alert to the user to accessory device 106 which may be, for example, a smartwatch, which may display the alert on user interface 178.

In another aspect of the invention, the equations for detecting the risk of hypoglycemia can be adjusted to determine the risk of hypoglycemia if the user wishes to engage in a particular activity, for example, exercise. Exercise tends to increase the user's sensitivity to insulin or lower the blood glucose levels of the user and as such, vigorous exercise may lower the blood glucose levels to a level that can be considered hypoglycemic. If the user is aware of the risk, the user may take steps to mitigate the risk prior to engaging in the activity.

In one embodiment, the same factors used to evaluate the risk of hypoglycemia or hyperglycemia may be used to evaluate the risk of hypoglycemia when the user wished to engage in exercise (eight risk factor) (or another activity). The risk factor based on the most current CGM reading can be evaluated as provided by Eq. (11), which provides a linear increase in the probability of hypoglycemia as a CGM decreases from a first threshold blood glucose value (for the eighth risk factor), e.g. 150 mg/dL to a second threshold blood glucose value (for the eighth risk factor), e.g. 100 mg/dL, with 100% probability when the blood glucose level is at the second threshold blood glucose value, e.g. 100 mg/dL. In some embodiments, the eighth risk factor is determined by assessing the negative deviation of the CGM reading value from the first threshold blood glucose value (for the eighth risk factor). The first threshold blood glucose value may be the target blood glucose value, or the lower value of a target blood glucose value range. In some embodiments, the eighth risk factor is determined by assessing the positive deviation of the CGM reading value from a hypoglycemia threshold value. The hypoglycemia threshold value may be the numerical value at which the user is considered as being hypoglycemic. In some embodiments, the eighth risk factor is determined by assessing where the CGM reading value is between the hypoglycemia threshold value and the first threshold blood glucose value, wherein the eighth risk factor is determined to be 0% when the CGM reading value is at or above the first threshold blood glucose value, the eighth risk factor is determined to be 100% when the CGM reading value is at or below the hypoglycemia threshold value, and wherein CGM reading values between the first threshold blood glucose value and the hypoglycemia threshold have a risk factor between 0% to 100%. In some embodiments, the CGM reading values between the first threshold blood glucose value and the hypoglycemia threshold are attributed a risk between 0% to 100% based on a linear increase the closer the CGM reading values are to the hypoglycemia threshold value. For example, for a first threshold blood glucose value at 150 mg/dL and a hypoglycemia threshold at 70 mg/dL, every negative deviation of 8 mg/dL from the first threshold blood glucose value may result in the eighth risk factor increasing by 10%, e.g. 134 mg/dL would result in the eighth risk factor being 20%. In some embodiments, the correlation between the eighth risk factor, the first threshold blood glucose value and the hypoglycemia threshold value may be non-linear. Other blood glucose values than those may be used. Using the exemplary values mentioned above, the probability can be expressed as follows:

$$P(i)_{CGM\,Value} = min\left(1, max\left(0, \frac{180 - CGM(i)}{80}\right)\right)$$

$$(11)$$

Accordingly, in some embodiments, the eighth risk factor is determined by the following steps: calculating a first value(for the eighth risk factor) as a first threshold blood glucose value (for the eighth risk factor) subtracted by the value of the CGM reading received during the current cycle of MDA 129, and

calculating a second value (for the eighth risk factor) by dividing the first value by a second threshold blood glucose value (for the eighth risk factor),
determining a third value (for the eighth risk factor), wherein the third value is set to 0 if the second value is smaller than 0, and wherein the third value is set to the second value if the second value is greater than 0,
determining the eighth risk factor to be 1 if the third value is greater than 1, and determining the eighth risk factor to be the third value if the third value is smaller than 1.

In some embodiments, the first threshold blood glucose value is preset by the user or a healthcare provider. In some embodiments, the first threshold blood glucose value is between about 150 mg/dL to about 230 mg/dL, more specifically between about 160 mg/dL to about 210 mg/dL, and in particular between about 170 mg/dL to about 190 mg/dL. In some embodiments, the first threshold blood glucose value is the first threshold value used to determine the first risk factor increased by an exercise factor, in particular wherein the exercise factor accounts for an expected or average drop of blood glucose due to exercise. In some embodiments, the second threshold blood glucose value is preset by the user or healthcare provider. In some embodiments, the second threshold blood glucose value is between about 50 mg/dL to about 130 mg/dL, more specifically between about 60 mg/dL to about 110 mg/dL, and in particular between about 70 mg/dL to about 90 mg/dL. In some embodiments, the second threshold blood glucose value is calculated as the first threshold blood glucose value subtracted by a hypoglycemia threshold value.

As can be seen, in Eq. (11), if as the CGM reading for the current cycle approaches 100 mg/dL, the fraction in Eq. (11) becomes equal to 1, yielding a 100% probability for this factor. It should be noted that, in this example, Eq. (11) is identical to Eq. (1), except for the constant "180", which has been increased from the constant "150" from Eq. (1) to account for the effect of the exercise on the use blood glucose level. This is based on the assumption that exercise will lower the user's blood glucose level by as much as 30 mg/dL. Other values may be used.

A second factor that may be used for determining the risk of hypoglycemia in the presence of exercise, which is based on the CGM trend, can be calculated using Eq. (2), which indicates a linear increase in the probability of hypoglycemia as a rate of change of use blood glucose in the previous 15 minutes gets closer to -60 mg/dL, or a decrease in 20 mg/dL per cycle, with 100% probability when the rate of change is -20 mg/dL per cycle or worse. Other values may be used.

An insulin on board factor (ninth risk factor), taking into account the effect of exercise, may be calculated by Eq. (12) below, which provides for a linear increase in the probability to 100% if the current insulin-on-board is sufficient to bring user's blood glucose level to a first threshold blood glucose value (for the ninth risk factor), e.g. 40 mg/dL, and 0% if the current insulin-on-board is sufficient to bring user's blood glucose level to a second threshold value (for the ninth risk factor), e.g. 180 mg/dL. Other blood glucose values may be used. Using these exemplary values, the probability can be expressed as follows:

$$P(i)_{IOB} = min\left(1, max\left(0, \frac{CF(i)IOB(i) - (CGM(i) - 180)}{140}\right)\right)$$

(12)

Accordingly, in some embodiments, the ninth risk factor based on the insulin-on-board is determined by the following steps:

calculating a first value (for the ninth risk factor) by multiplying the user's correction factor for cycle $i$ with the insulin-on-board for cycle $i$,
calculating a second value by subtracting the second threshold value (for the ninth risk factor) from the current blood glucose reading for cycle $i$ (of MDA 129),
calculating a third value by subtracting the second value from the first value,
calculating a fourth value by subtracting the first blood glucose threshold value (for the ninth risk factor) from the second threshold value,
calculating a fifth value by dividing the third value by the fourth value,
determining a sixth value, wherein the sixth value is set to 0 if the fifth value is smaller than 0, and wherein the sixth value is set to the fifth value if the fourth value is greater than 0,
determining the ninth risk factor based on the insulin-on-board to be 1 if the sixth value is greater than 1, and determining the ninth risk factor to be the sixth value if the sixth value is smaller than 1.

A personalization factor for determining the risk of hypoglycemia in the presence of exercise may calculated by Eq. (4) and the decision whether to issue an alert may be determined by Eq. (5). The process for determining the risk of hypoglycemia in the presence of exercise or other activities is provided by the flowchart in **FIG. 2.**

In one embodiment, the risk of hypoglycemia in the presence of a particular activity may be evaluated only at the request of the user, typically prior to the user engaging in the activity or exercise. In other instances, if the user has a regular schedule of exercise, the risk may be evaluated periodically and a constant status may be provided to the user.

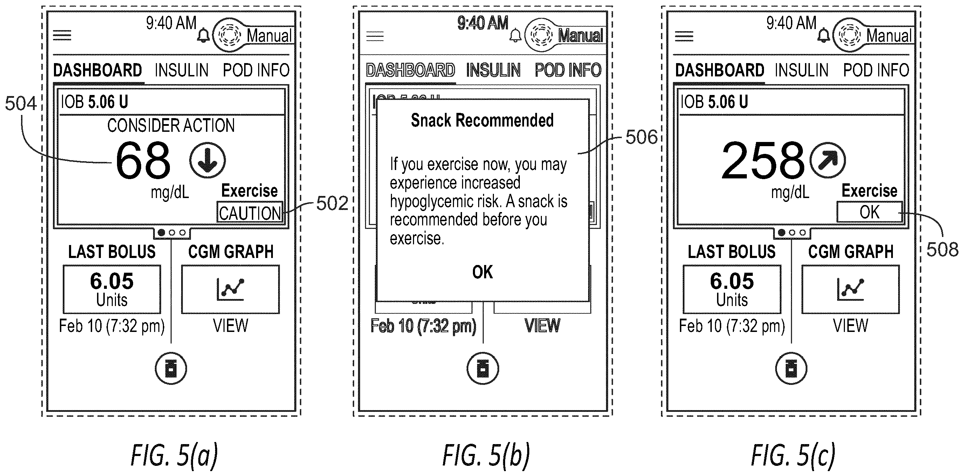

**FIG. 5(a)** shows exemplary text flag 502 providing an alert to the user indicating that user should be cautious when engaging in exercise, based on the factors previously described. The text flag 502 may be displayed in a particular color, for example, red, so as to bring the user's attention to the warning. In addition, the user's most recent blood glucose level 504 is shown, which may also be shown in red, with the downward arrow indicating a downward trend in the user's blood glucose levels. In addition, as shown in **FIG. 5(b),** a modal window 506 may be displayed indicating that the user should ingest a quantity of carbohydrates before engaging in the activity or exercise to mitigate the risk of hypoglycemia. The user may dismiss the modal window 506 by tapping the "OK" button. Alternatively, exemplary modal window 506 may be dismissed automatically after a predetermined period of time. Modal window 506 may be used in lieu of or in addition to text flag 502 shown in **FIG. 5(a). FIG. 5(c)** shows text flag 508 indicating that there is little or no danger to the user at the present time if the user engages in exercise, or an activity that would, under other circumstances, cause the use blood glucose level to fall to a level where the user would be considered hypoglycemic.

As with the previous alerts, the text flag 502 and/or the modal window 506 may be displayed on the default screen of user app 160 on user device 105. Alternatively, in the absence of user device 105, the drug delivery device 102 may provide, visual, audible or haptic feedback via its user interface 127 as previously described. In addition, the warning may be communicated to accessory device 106, for example, smartwatch, where the alert may be displayed in user interface 178. Additionally, as before, the actual text and colors of the text flag 502 and the modal window 506 are exemplary only and are not meant to limit the scope of the invention.

In yet a third aspect of the invention, the embodiments described herein may be assisted by a machine learning model. The machine learning model may be trained to provide a weighting factor for each risk factor depending upon a correlation over time with actual outcomes. In one example, a matrix of each indicator for each alert can be compared against actual outcomes of those alerts. The indicator that has the greatest correlation with hyperglycemic or hypoglycemic outcomes can be provided with a higher weight (e.g., a larger coefficient may be applied to such indicator(s) *vis a vis* the other indicator(s)). The outcome of each indicator can also be shared across a population of multiple users, with the indicator that best correlates with the actual hyperglycemic or hypoglycemic outcomes across the most number of users providing a heavier weight for new users of the system.

As would be realized by one of skill in the art, many variations on the embodiments disclosed herein are possible. In particular, various sizes, materials and configurations are contemplated to be the within the scope of the invention and the invention is not meant to be limited by the specific embodiments disclosed herein

The following examples pertain to various embodiments disclosed herein for the needle insertion/reduction mechanism for use with an automatic drug delivery system.

Example 1 is a first embodiment of the invention is directed to a method for alerting the user of an automatic drug delivery system of a risk of a hypoglycemic or hyperglycemic condition comprising receiving an indication of a current blood glucose level of the user, calculating one or more factors based on the current blood glucose level the user indicative of the risk of a hypoglycemic or hyperglycemic condition and providing an alert to the user via a user interface of the automatic drug delivery system when the risk is indicated based on the one or more factors.

Example 2 is an extension of Example 1, or any other example disclosed herein, wherein the one or more risk factors include a factor indicating a linear relationship between a probability of hypoglycemia and a drop in the blood glucose level of the user or the probability of hyperglycemia and a rise in the blood glucose level of the user.

Example 3 is an extension of Example 2, or any other example disclosed herein, wherein the one or more risk factors include a factor indicating a linear relationship between a probability of hypoglycemia and a rate of change of the blood glucose level the user below a predetermined threshold per unit time or probability of hyperglycemia and a rate of change of the blood glucose level of the user above a predetermined threshold per unit of time.

Example 4 is an extension of Example 3, or any other example disclosed herein, wherein the one or more risk factors include a factor indicating a linear relationship between a probability of hypoglycemia and an amount of insulin-on-board the user necessary to bring the blood glucose level of the user to zero or a probability of hyperglycemia and amount of insulin-on-board the user necessary to bring the blood glucose level of the user to a predetermined threshold.

Example 5 is an extension of Example 4, or any other example disclosed herein, the method further comprising calculating an additional risk factor based on the accuracy of previous alerts issued to the user.

Example 6 is an extension of Example 5, or any other example disclosed herein, the method further comprising combining the risk factors to determine whether the alert should be provided to the user.

Example 7 is an extension of example 6, or any other example disclosed herein, wherein the alert comprises a text flag displayed on the screen of a user application running on a user device portion of the automatic drug delivery system.

Example 8 is an extension of Example 6, or any other example disclosed herein, wherein the alert comprises a modal window displayed on a screen of the user application running on a user device portion of the automatic drug delivery system.

Example 9 is an extension of Example 6, or any other example disclosed herein, wherein the alert comprises a visual, audible or haptic feedback provided via a user interface of wearable drug delivery device portion of the automatic drug delivery system.

Example 10 is an extension of Example 6, or any other example disclosed herein, the method further comprising a machine learning model providing weighting factors applicable to the risk factors, the weighting factors being dependent upon the accuracy of previous alerts.

Example 11 is an extension of Example 10, or any other example disclosed herein, wherein the machine learning model is aggregated over large population of users to provide weighting factors for new users of the automatic drug delivery system.

Example 12 is an extension of Example 1, or any other example disclosed herein, wherein the method is executed periodically or each time a new blood glucose reading for the user is received.

Example 13 is an extension of claim 1, or any other example disclosed herein, wherein the risk of hypoglycemia is determined based on the assumption that the user intends to engage in a specific activity.

Example 14 is an extension of Example 13, or any other example disclosed herein, wherein the assessment of the risk of hypoglycemia in the presence of the activity is calculated at the request of the user.

Example 15 is a second embodiment of the invention directed to a system comprising a drug delivery device, a blood glucose sensor in communication with the drug delivery device, a user device in communication with the drug delivery device and software executing on the user device or on the drug delivery device which causes the system to receive an indication of a current blood glucose level of the user, calculate one or more factors based on the current blood glucose level of the user indicative of the risk of a hypoglycemic or hyperglycemic condition and provide an alert to the user via a user interface of the automatic drug delivery system when the risk is indicated based on the one or more factors.

Example 16 is an extension of Example 15, or any other example disclosed herein, wherein the one or more factors includes a factor indicating a linear relationship between the probability of hypoglycemia and a drop in the blood glucose level of the user or the probability of hyperglycemia and a rise in the blood glucose level the user, a factor indicating a linear relationship between a probability of hypoglycemia and a rate of change in the blood glucose level of the user below a predetermined threshold per unit time or a probability of hyperglycemia and a rate of change in the blood glucose level of the user above a predetermined threshold per unit time and a factor indicating a linear relationship between a probability of hypoglycemia and amount of insulin-on-board the user necessary to bring the blood glucose level of the user to 0 or probability of hyperglycemia and amount of insulin-on-board the user necessary to bring the blood glucose level of the user to a predetermined threshold.

Example 17 is an extension of Example 16, or any other example disclosed herein, wherein the software further causes the system to calculate an additional risk factor based on the accuracy of previous alerts issued to the user.

Example 18 is an extension of Example 17, or any other example disclosed herein, wherein the software further causes the system to combine the risk factors to determine whether the alert should be provided to the user.

Example 19 is extension of Example 18, or any other example disclosed herein, wherein the alert comprises a text flag or modal window displayed on the screen of the user device.

Example 20 is extension of Example 18, or any other example disclosed herein, wherein the alert comprises a visual, audible or haptic feedback provided via the drug delivery device.

Software related implementations of the techniques described herein may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

To those skilled in the art to which the invention relates, many modifications and adaptations of the invention may be realized. Implementations provided herein, including sizes, shapes, ratings, compositions and specifications of various components or arrangements of components, and descriptions of specific manufacturing processes, should be considered exemplary only and are not meant to limit the invention in any way. As one of skill in the art would realize, many variations on implementations discussed herein which fall within the scope of the invention are possible. Moreover, it is to be understood that the features of the various embodiments described herein were not mutually exclusive and can exist in various combinations and permutations, even if such combinations or permutations were not made express herein, without departing from the spirit and scope of the invention. Accordingly, the method and apparatus disclosed herein are not to be taken as limitations on the invention but as an illustration thereof. The scope of the invention is defined by the claims which follow.

Although the present invention is defined in the attached claims, it should be understood that the present invention can also (alternatively) be defined in accordance with the following embodiments:

1. A method for alerting a user of an automated drug delivery system of a risk of a hypoglycemic or hyperglycemic condition comprising:

   receiving an indication of a current blood glucose level of the user;
   calculating one or more factors based on the current blood glucose level of the user indicative of a risk of a hypoglycemic or hyperglycemic condition; and
   providing an alert to the user via a user interface of the automatic drug delivery system when the risk is indicated based on the one or more factors.

2. The method of embodiment 1 wherein the one or more risk factors include a factor indicating a linear relationship between a probability of hypoglycemia and a drop in the blood glucose level of the user or the probability of hyperglycemia and a rise in the blood glucose level of the user.

3. The method of embodiment 2 wherein the one or more risk factors include a factor indicating a linear relationship between a probability of hypoglycemia and a rate of change of the blood glucose level of the user below a predetermined threshold per unit time or a probability of hyperglycemia and a rate of change of the blood glucose level of the user above a predetermined threshold per unit time.

4. The method of embodiment 3 wherein the one or more risk factors include a factor indicating a linear relationship between a probability of hypoglycemia and an amount of insulin-on-board the user necessary to bring the blood glucose level of the user to zero or a probability of hyperglycemia and an amount of insulin-on-board the user necessary to bring the blood glucose level of the user to a predetermined threshold.

5. The method of embodiment 4 further comprising:
calculating an additional risk factor based on accuracy of previous alerts issued to the user.

6. The method of embodiment 5 further comprising:
combining the risk factors to determine whether the alert should be provided to the user.

7. The method of embodiment 6, wherein the alert comprises a text flag displayed on a screen of a user application running on a user device portion of the automatic drug delivery system.

8. The method of embodiment 6 wherein the alert comprises a modal window displayed on a screen of a user application running on a user device portion of the automatic drug delivery system.

9. The method of embodiment 6 wherein the alert comprises a visual, audible or haptic feedback provided via a user interface of a wearable drug delivery device portion of the automatic drug delivery system.

10. The method of embodiment 6 further comprising:
a machine learning model providing weighting factors applicable to the risk factors, the weighting factors being dependent upon the accuracy of previous alerts.

11. The method of embodiment 10 were in the machine guarding model is aggregated over a large population of users to provide weighting factors for new users of the automatic drug delivery system.

12. The method of embodiment 1 wherein the method is executed periodically or each time a new blood glucose reading for the user is received.

13. The method of embodiment 1 wherein the risk of hypoglycemia is determined based on the assumption that the user intends on engaging in a specific activity.

14. The method of embodiment 13 wherein the assessment of the risk of hypoglycemia in the presence of the activity is calculated at the request of the user.

15. A system comprising:

a drug delivery device;
a blood glucose sensor in communication with the drug delivery device;
a user device in communication with the drug delivery device; and
software executing on the user device or on the drug delivery device which causes the system to:

receive an indication of a current blood glucose level of the user;
calculate one or more factors based on the current blood glucose level of the user indicative of a risk of a hypoglycemic or hyperglycemic condition; and
provide an alert to the user via a user interface of the automatic drug delivery system when the risk is indicated based on the one or more factors.

16. The system of embodiment 15 wherein the one or more risk factors includes:

a factor indicating a linear relationship between a probability of hypoglycemia and a drop in the blood glucose level of the user or the probability of hyperglycemia and a rise in the blood glucose level of the user;
a factor indicating a linear relationship between a probability of hypoglycemia and a rate of change of the blood glucose level of the user below a predetermined threshold per unit time or a probability of hyperglycemia and a rate of change of the blood glucose level of the user above a predetermined threshold per unit time; and
a factor indicating a linear relationship between a probability of hypoglycemia and an amount of insulin-on-board the user necessary to bring the blood glucose level of the user to zero or a probability of hyperglycemia and an amount of insulin-on-board the user necessary to bring the blood glucose level of the user to a predetermined threshold.

17. The system of embodiment 16, the software further causing the system to:
calculate an additional risk factor based on the accuracy of previous alerts issued to the user.

18. The system of embodiment 17, the software further causing the system to:
combine the risk factors to determine whether the alert should be provided to the user.

19. The system of embodiment 18, wherein the alert comprises a text flag or modal window displayed on a screen of the user device.

20. The system of embodiment 18 wherein the alert comprises a visual, audible or haptic feedback provided via the drug delivery device.

**Claims**

1. A method for alerting a user of an automated drug delivery system of a risk of a hypoglycemic or hyperglycemic condition comprising:

   receiving an indication of a current blood glucose level of the user;
   calculating one or more factors based on the current blood glucose level of the user indicative of a risk of a hypoglycemic or hyperglycemic condition; and
   providing an alert to the user via a user interface of the automatic drug delivery system when the risk is indicated based on the one or more factors.

2. The method of claim 1, the risk factor is based on a deviation of the current blood glucose level of the user from a first threshold blood glucose value, more specifically wherein the risk factor is determined by assessing where the current blood glucose level of the user is between the first threshold value and a second threshold blood glucose value, wherein the second threshold blood glucose value is based on a hyper- or hypoglycemia threshold value, in particular wherein current blood glucose levels of the user between the first threshold blood glucose value and the second threshold blood glucose value are attributed a risk factor between 0% to 100% based on a linear increase the closer the current blood glucose values are to the second threshold blood glucose value.

3. The method according to any preceding claim, wherein the risk factor is based on a rate of change of the current blood glucose level of the user, more specifically the risk factor may be based on the rate of change of the current blood glucose level of the user towards a hypoglycemia threshold value or hyperglycemia threshold value, even more specifically wherein the risk factor is based on a comparison of the rate of change of the current blood glucose level of the user to a rate of change threshold value and in particular wherein rates of change between 0 and the rate of change threshold value are attributed a risk factor between 0% to 100% based on a linear increase the closer the rate of change is to the rate of change threshold value.

4. The method according to any preceding claim, wherein the risk factor is based on a current insulin-on-board, more specifically the third risk factor increases with increasing insulin-on-board, in particular relative to the current blood glucose reading of the user.

5. The method of any preceding claim further comprising:
   calculating an additional risk factor based on accuracy of previous alerts issued to the user.

6. The method of any preceding claim further comprising:
   combining the risk factors to determine whether the alert should be provided to the user.

7. The method of any preceding claim, wherein the alert comprises a text flag displayed on a screen of a user application running on a user device portion of the automatic drug delivery system, wherein the alert comprises a modal window displayed on a screen of a user application running on a user device portion of the automatic drug delivery system, and/or wherein the alert comprises a visual, audible or haptic feedback provided via a user interface of a wearable drug delivery device portion of the automatic drug delivery system.

8. The method of any preceding claim further comprising:
   a machine learning model providing weighting factors applicable to the risk factors, the weighting factors being dependent upon the accuracy of previous alerts.

9. The method of any preceding claim, wherein the machine learning model is aggregated over a large population of users to provide weighting factors for new users of the automatic drug delivery system.

10. The method of any preceding claim wherein the method is executed periodically or each time a new blood glucose reading for the user is received, wherein the risk of hypoglycemia is determined based on the assumption that the user intends on engaging in a specific activity, and/or wherein the assessment of the risk of hypoglycemia in the presence of the activity is calculated at the request of the user.

11. A system comprising:

a drug delivery device;
a blood glucose sensor in communication with the drug delivery device;
a user device in communication with the drug delivery device; and
software executing on the user device or on the drug delivery device which causes the system to:

receive an indication of a current blood glucose level of the user;
calculate one or more factors based on the current blood glucose level of the user indicative of a risk of a hypoglycemic or hyperglycemic condition; and
provide an alert to the user via a user interface of the automatic drug delivery system when the risk is indicated based on the one or more factors.

12. The system of claim 11 wherein the one or more risk factors includes:

a factor indicating a linear relationship between a probability of hypoglycemia and a drop in the blood glucose level of the user or the probability of hyperglycemia and a rise in the blood glucose level of the user;
a factor indicating a linear relationship between a probability of hypoglycemia and a rate of change of the blood glucose level of the user below a predetermined threshold per unit time or a probability of hyperglycemia and a rate of change of the blood glucose level of the user above a predetermined threshold per unit time; and
a factor indicating a linear relationship between a probability of hypoglycemia and an amount of insulin-on-board the user necessary to bring the blood glucose level of the user to zero or a probability of hyperglycemia and an amount of insulin-on-board the user necessary to bring the blood glucose level of the user to a predetermined threshold.

13. The system of claim 11 or 12, the software further causing the system to:
calculate an additional risk factor based on the accuracy of previous alerts issued to the user.

14. The system of any one of claims 11 to 13, the software further causing the system to:
combine the risk factors to determine whether the alert should be provided to the user.

15. The system of any one of claims 11 to 14, wherein the alert comprises a text flag or modal window displayed on a screen of the user device and/or wherein the alert comprises a visual, audible or haptic feedback provided via the drug delivery device.

FIG. 1

EP 4 300 514 A1

Receive Blood
Glucose Reading for
Current Cycle
202

Calculate
Probability based on
Current CGM
Reading
204

Calculate
Probability Based
on CGM Trend
206

Calculate
Probability Based
on IOB
208

Calculate Accuracy
of last *k* alerts.
210

Should Alert
Be
Triggered?
212

Yes

No

Trigger Alert
214

Wait for Next Cycle
216

*FIG. 2*

FIG. 3(a)

FIG. 3(b)

FIG. 3(c)

EP 4 300 514 A1

**FIG. 4(a)**

**FIG. 4(b)**

**FIG. 4(c)**

FIG. 5(a)          FIG. 5(b)          FIG. 5(c)

EP 4 300 514 A1

EP 4 300 514 A1

## EUROPEAN SEARCH REPORT

Application Number

EP 23 18 2770

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/289821 A1 (RACK-GOMER ANNA LEIGH [US] ET AL) 15 October 2015 (2015-10-15)<br>* paragraph [0007] *<br>* paragraph [0009] *<br>* paragraph [0011] *<br>* paragraph [0056] *<br>* paragraph [0146] – paragraph [0147] *<br>* paragraph [0151] *<br>* paragraph [0165] *<br>* paragraph [0194] *<br>* paragraph [0259] *<br>* table II * | 1-8,10, 11,13-15 | INV.<br>G16H50/30<br>A61B5/145<br>A61B5/00<br>A61M5/172<br>G16H20/17<br>G16H40/60<br>G16H50/20 |
| X | WO 2021/007485 A1 (UNIV VIRGINIA PATENT FOUNDATION [US]) 14 January 2021 (2021-01-14)<br>* paragraph [0023] *<br>* paragraph [0027] *<br>* paragraph [0031] – paragraph [0035] *<br>* paragraph [0040] – paragraph [0041] *<br>* paragraph [0068] *<br>* paragraph [0075] *<br>* figure 1 * | 1,3-9, 11-15 | |
| X | US 2018/055452 A1 (BRETON MARC D [US]) 1 March 2018 (2018-03-01)<br>* paragraph [0024] *<br>* paragraph [0026] *<br>* paragraph [0032] *<br>* paragraph [0038] *<br>* paragraph [0062] *<br>* figure 3 * | 1,3,4,6, 10-12,14 | TECHNICAL FIELDS SEARCHED (IPC)<br>G16H<br>A61B<br>A61M |

–/––

The present search report has been drawn up for all claims

| Place of search | Date of completion of the search | Examiner |
|---|---|---|
| Munich | 13 October 2023 | Bonnet, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt

European Patent Office

Office européen des brevets

# EUROPEAN SEARCH REPORT

Application Number

EP 23 18 2770

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/057042 A1 (HAYTER GARY [US]) 4 March 2010 (2010-03-04) * paragraph [0045] * * paragraph [0051] * * paragraph [0083] * * paragraph [0089] * ----- | 1,3-7, 11,13-15 | |
| A | WO 2018/077835 A1 (NOVO NORDISK AS [DK]) 3 May 2018 (2018-05-03) * claims 1-14 * ----- | 1-15 | |

TECHNICAL FIELDS SEARCHED (IPC)

The present search report has been drawn up for all claims

| Place of search | Date of completion of the search | Examiner |
|---|---|---|
| Munich | 13 October 2023 | Bonnet, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 18 2770

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2023

| Patent document cited in search report | | Publication date | Patent family member(s) | | Publication date |
|---|---|---|---|---|---|
| US 2015289821 | A1 | 15-10-2015 | AU | 2015244291 A1 | 04-08-2016 |
| | | | AU | 2017225153 A1 | 05-10-2017 |
| | | | AU | 2019200136 A1 | 31-01-2019 |
| | | | AU | 2021202444 A1 | 13-05-2021 |
| | | | AU | 2022287585 A1 | 02-02-2023 |
| | | | CA | 2936774 A1 | 15-10-2015 |
| | | | CN | 106415556 A | 15-02-2017 |
| | | | CN | 111968737 A | 20-11-2020 |
| | | | EP | 3128913 A1 | 15-02-2017 |
| | | | EP | 3434184 A1 | 30-01-2019 |
| | | | EP | 3437558 A1 | 06-02-2019 |
| | | | JP | 7043531 B2 | 29-03-2022 |
| | | | JP | 7297964 B2 | 26-06-2023 |
| | | | JP | 2017515520 A | 15-06-2017 |
| | | | JP | 2020078648 A | 28-05-2020 |
| | | | JP | 2022079506 A | 26-05-2022 |
| | | | JP | 2023134431 A | 27-09-2023 |
| | | | US | 2015289821 A1 | 15-10-2015 |
| | | | US | 2015289823 A1 | 15-10-2015 |
| | | | WO | 2015156965 A1 | 15-10-2015 |
| WO 2021007485 | A1 | 14-01-2021 | AU | 2020309580 A1 | 10-02-2022 |
| | | | CA | 3146849 A1 | 14-01-2021 |
| | | | EP | 3996590 A1 | 18-05-2022 |
| | | | US | 2022257199 A1 | 18-08-2022 |
| | | | WO | 2021007485 A1 | 14-01-2021 |
| US 2018055452 | A1 | 01-03-2018 | US | 2018055452 A1 | 01-03-2018 |
| | | | US | 2020015757 A1 | 16-01-2020 |
| | | | US | 2023181123 A1 | 15-06-2023 |
| US 2010057042 | A1 | 04-03-2010 | US | 2010057042 A1 | 04-03-2010 |
| | | | US | 2014235981 A1 | 21-08-2014 |
| | | | WO | 2010025432 A1 | 04-03-2010 |
| WO 2018077835 | A1 | 03-05-2018 | NONE | | |

EPO FORM P0459

For more details about this annex : see Official Journal of the European Patent Office, No. 12/82

## REFERENCES CITED IN THE DESCRIPTION

**Patent documents cited in the description**

- US 7303549 B
- US 7137964 B

- US 6740059 B